# EUROPÄISCHE PATENTANMELDUNG

(11) **EP 2 839 815 A1**
(43) Veröffentlichungstag der Anmeldung: **25.02.2015**
(21) Anmeldenummer: 14178521.2
(22) Anmeldetag: 25.07.2014
(51) Int. Cl.: A61F 2/966

(54) **Kathetersystem mit beweglicher Hülle**

(30) Priorität: 20.08.2013 US 201361867626 P
(71) Anmelder: Biotronik AG, 8180 Bülach (CH)
(72) Erfinder: Hepke, Markus, 8006 Zürich (CH); Wesselmann, Matthias, 8455 Rüdlingen (CH); Eckermann, Fabian, 8484 Weisslingen (CH)
(74) Vertreter: Galander, Marcus

(57) **Zusammenfassung**

Die Erfindung beschreibt ein Kathetersystem zum Einbringen eines selbstexpandierenden Stents in ein Körpergefäß. Der Katheter 1 weist eine bewegliche Hülle 30 auf, die den selbstexpandierenden Stent im Raum 2 bei Positionierung im Körpergefäß hält. Beim Freisetzen des selbstexpandierenden Stents wird die Hülle durch Fluidbeaufschlagung des Ballons 20 zurück geschoben. Die bewegliche Hülle 30 kann dabei präzise durch Einstellung des Druckverhältnisses in den Ballonen 20, 23 nach proximal oder distal bewegt werden. Entsprechend sind eine kontrollierte Freisetzung und das erneute Einfangen des selbstexpandierenden Stents möglich.

## Beschreibung

Die Erfindung betrifft ein Kathetersystem umfassend einen Katheter mit einem funktionalen Bauteil im distalen Bereich,
- wobei das Kathetersystem zum Einbringen in ein Körpergefäß geeignet ist,
- wobei der Katheter an seinem distalen Bereich eine bewegliche Hülle aufweist, deren Durchmesser zumindest dem Durchmesser des funktionalen Bauteils entspricht, und deren Länge größer als die Länge des funktionalen Bauteils ist,
- wobei die Hülle das funktionale Bauteil überdeckt, wenn das Kathetersystem in dem bestimmungsgemäßen Zustand ist, in dem der Katheter in das Körpergefäß eingeführt wird,
- wobei der Katheter zumindest im distalen Bereich einen Schaft mit einem ersten inneren Lumen und ein äußeres zweites Lumen aufweist.

Katheter, insbesondere drahtgeführte Katheter, haben eine breite Verwendung bei vorwiegend medizinischen Anwendungen und Eingriffen gefunden. Insbesondere in der Angioplastie und bei der Implantation von Stents werden drahtgeführte Katheter eingesetzt. Bei derartigen Anwendungen wird der Katheter mit Hilfe eines vorher in den Körper des Patienten eingeführten Führungsdrahts positioniert. Die distale Spitze eines Katheter-Führungsdrahts ist in der Regel sehr flexibel ausgebildet, so dass diese gebogen und gedreht werden kann, um zu der gewünschten Stelle im Körper des Patienten, z. B. innerhalb eines Blutgefäßes, vorgeschoben werden zu können. Es sind verschiedenste drahtgeführte Kathetertypen bekannt, so beispielsweise interventionelle Katheter, Ballonkatheter und Katheter zur Applikation von selbstexpandierenden Stents.

Mittels interventioneller Katheter lässt sich ein breites Spektrum der so genannten interventionellen Kardiologie oder Radiologie abdecken. Dies reicht von vaskulären Interventionen (wie beispielsweise Angioplastie von Gefäßen oder der Einbau von Filtern in Körpergefäße) über tumorablative Interventionen, bei denen beispielsweise Tumore durch Kälte lokal zerstört werden, bis zu Interventionen unter Steuerung von bildgebenden Verfahren, wie beispielsweise eine Biopsie.

Die vorliegende Erfindung wird im Folgenden hauptsächlich am Beispiel der vaskulären Intervention, insbesondere an Kathetersystemen zum Einbringen von selbstexpandierenden Stents, beschrieben, ist jedoch nicht darauf beschränkt. Die Erfindung ist für alle Kathetersysteme mit funktionalen Bauteilen im distalen Bereich geeignet, bei denen die funktionalen Bauteile von einer beweglichen Hülle überdeckt werden müssen, wenn der Katheter in das jeweils zu behandelnde Körpergefäß an den Behandlungsort eingebracht wird.

Vaskuläre Interventionen bilden einen großen Bereich der interventionellen Radiologie. Häufig werden hierbei verschlossene Gefäße rekanalisiert und aufgeweitet. Zum Aufweiten von verschlossenen Körpergefäßen werden häufig Ballonkatheter verwendet. Ein derartiger Ballonkatheter hat einen Fluid befüllbaren Ballon an seinem distalen Ende. Dieser wird unter radiologischer Kontrolle in das Körpergefäß an die verengte Stelle geführt. Dort wird der Ballon durch die Beaufschlagung mittels Fluid expandiert und dadurch das umgebende Körpergefäß aufgeweitet.

Im Rahmen dieser Anmeldung wird unter dem proximalen Ende eines Katheters das Ende verstanden, welches in der Hand des Behandlers außerhalb des Körpers/des Körpergefäßes liegt. Das distale Ende eines Katheters ist entsprechend die in das Körpergefäß geführte Spitze des Katheters. Entsprechend sind die Lagebezeichnungen proximal (näher zum Behandler) und distal (näher zur im Körpergefäß befindlichen Spitze des Katheters) zu verstehen.

Im Rahmen dieser Anmeldung werden die Begriffe Expansion/expandieren, Dilatation/dilatieren und Aufdehnung/aufdehnen des Ballons oder eines Stents als Synonym betrachtet, wobei im Rahmen dieser Anmeldung der Begriff Expansion/expandieren verwendet wird.

Um eine erneute Verengung aufgeweiteter Gefäße zu vermeiden, wird häufig ein Stent an die betroffene Stelle in das Körpergefäß eingebracht. Ein Stent ist zumeist eine kleine gitterförmige Struktur in einer Form ähnlich eines Röhrchens aus Metall oder Kunststoff. Ein derartiger Stent kann entweder mit einem Ballonkatheter oder mit einem Katheter zur Applikation selbstexpandierender Stents in das Körpergefäß eingebracht werden.

Bei der Verwendung eines Ballonkatheters wird der Stent auf den Ballon gebracht (im Stand der Technik häufig als "gecrimpt" bezeichnet) und dort in geeigneter Weise befestigt. Durch die Expansion des Ballons an der Stelle der Verengung wird der Stent expandiert und verbleibt nach Druckentlastung und entsprechender Rückfaltung des Ballons im Körpergefäß zur Stützung. Die Aufweitung des Gefäßes und die Platzierung des Stents können dabei in einem oder in aufeinander folgenden Schritten durchgeführt werden.

Selbstexpandierende Stents bestehen aus einem Formgedächtnismaterial, insbesondere spezielle Metalllegierungen wie die als Nitinol bekannte Nickel-Titan Legierung. Der selbstexpandierende Stent hat bei Körpertemperatur die gewünschte Form, die zur Stützung des Körpergefäßes notwendig ist. Er wird bei geringerer Temperatur auf einen Katheter zur Applikation selbstexpandierender Stents gebracht, komprimiert und dort mit einem geeigneten Mittel in einer komprimierten Form gehalten. Am Ort der Applikation im Körpergefäß werden diese Mittel entfernt und der selbstexpandierende Stent nimmt seine ursprüngliche Form ein und stützt das Gefäß.

Selbstexpandierende Stents sind auch zur Implantation von künstlichen Herzklappen bekannt. Hier werden die künstlichen Herzklappen direkt mit dem selbstexpandierenden Stent vernäht. Anschließend wird der selbstexpandierende Stent mit der Herzklappe, ähnlich wie vorangehend geschildert, im Körper platziert.

Die vorliegende Erfindung beschreibt ein Kathetersystem mit einem Katheter mit einem funktionalen Bauteil im distalen Bereich und ist nicht auf selbstexpandierende Stents zur Behandlung von Verengungen eines Körpergefäßes oder selbstexpandierende Stents mit künstlichen Herzklappen beschränkt, jedoch insbesondere für diese Anwendungen geeignet.

Bei der Applikation eines selbstexpandierenden Stents befindet sich der Führungsdraht, welcher mit Röntgenmarkern ausgestattet oder komplett unter Röntgenstrahlung sichtbar ist, schon im Körpergefäß. Über diesen Führungsdraht wird der Katheter mit der Spitze voran eingeführt. Die Spitze des Katheters ist dabei sehr flexibel, um Verletzungen der Wände des Körpergefäßes zu vermeiden, und derart beschichtet, dass ein möglichst reibungsfreies Gleiten im Körpergefäß ermöglicht wird. Zusätzlich ist sie sichtbar unter Röntgenstrahlung. Der Führungsdraht wird im ersten inneren Lumen des Katheters geführt.

Im Rahmen dieser Anmeldung wird das Lumen für den Führungsdraht immer als inneres Lumen (und entsprechend innerer Schaft) bezeichnet, unabhängig von koaxialer oder paralleler Anordnung weiterer Lumen.

Der Katheter wird mit Hilfe des Führungsdrahtes so platziert, dass der Stent an der gewünschten Position im Gefäß ist. Um eine sichere Platzierung zu gewährleisten, weißt der Schaft oder die bewegliche Hülle entsprechende Röntgenmarkierungen auf. Durch die bewegliche Hülle ist der selbstexpandierende Stent auf dem Katheter fixiert. Durch eine entsprechende Wegbewegung der Hülle nimmt der selbstexpandierende Stent seine ursprüngliche Form an und wird im Körpergefäß freigesetzt bzw. implantiert.

Zum Freisetzen eines selbstexpandierenden Stents sind im Stand der Technik verschiedene Lösungen bekannt. Im einfachsten Fall ist die bewegliche Hülle über den Stent als Schlauch ausgeführt, der den Katheter von außen umschließt. Zum Freisetzen des Stents wird dieser Außenschlauch einfach zurückgezogen. Diese Lösung ist vor allem bei so genannten Over-the-Wire Kathetern verbreitet. Bei einem Over-the-Wire Kathetersystem ist das innere Lumen für den Führungsdraht koaxial angeordnet. Im inneren Schaft mit dem inneren Lumen befindet sich der Führungsdraht, auf diesen befindet sich am distalen Ende der selbstexpandierende Stent, welcher vom Außenschlauch als bewegliche Hülle umgeben ist.

EP 2 329 799 offenbart ein ähnliches Kathetersystem jedoch für einen so genannten Monorail-Katheter, auch als Rapid-Exchange-Katheter bezeichnet. Bei derartigen Kathetersystemen läuft der Führungsdraht im Gegensatz zu Over-the-Wire-Kathetern nicht auf der gesamten Länge im koaxialen inneren Führungsdrahtlumen des Katheters entlang, sondern wird nahe dem distalen Ende des Katheters aus dem Führungsdrahtlumen herausgeführt. Zu diesem Zweck hat der Katheter an der entsprechenden Stelle einen Führungsdrahtausgang. Um bei einem derartigen System das Zurückziehen des Außenschlauchs zu gewährleisten, weist das in EP 2 329 799 beschriebene Kathetersystem eine Schneidvorrichtung für den Außenschlauch auf.

In den beiden geschilderten Ausbildungen des Standes der Technik wird die Bewegung der Hülle durch eine mechanische Kraftübertragung vermittelt. Der Außenschlauch wird mechanisch vom selbstexpandierenden Stent gezogen, die Haltekraft auf den Katheter entfällt dadurch und der Stent wird im Körpergefäß freigesetzt.

Ein anderes System der Freisetzung eines selbstexpandierenden Stents wird in WO 2006/089517 beschrieben. Hier erfolgt die Übertragung der Kraft zur Freisetzung des Stents hydraulisch. Dabei wird entweder der selbstexpandierende Stent mittels eines Kolbens und hydraulischer Kraftübertragung aus der ihn umgebenden Hülle geschoben oder die den Stent umgebende Hülle mittels Kolben und hydraulischer Kraftübertragung nach proximal zurück gedrückt. Die bewegliche Hülle weist dabei eine größere Länge als der Stent auf und schließt an ihrem proximalen Ende mit einer ringförmigen Dichtung ab, die zum Innenschaft hin abdichtet. Am proximalen Ende des Stents befindet sich ebenfalls eine ringförmige Dichtung, so dass sich zwischen den beiden ringförmigen Dichtungen und der beweglichen Hülle ein Raum bildet, der mit einem Fluid über das innere Lumen des Innenschafts beaufschlagt werden kann. Bei Beaufschlagung dieses Raumes mit einem Fluid wird die bewegliche Hülle so einem Kolben gleich nach distal gedrückt und über den in Position bleibenden Stent weg bewegt. Der selbstexpandierende Stent wird derart im Körpergefäß freigesetzt.

Beide im Stand der Technik bekannte Kathetersysteme zum Freisetzen eines selbstexpandierenden Stents weisen Nachteile auf. Die mechanische Freisetzung des selbstexpandierenden Stents geht mit hohen Reibungskräften einher. Dies ist insbesondere der Fall, wenn der Stent in ein sehr gewundenes Körpergefäß frei gesetzt werden soll. Diese hohen Reibungskräfte und der damit beim Behandler nötige Kraftaufwand erschweren die präzise örtliche Freisetzung des Stents. Das in WO 2006/089517 offenbarte Kathetersystem mit der hydraulischen Freisetzung des Stents ist durch die notwendigen Ringdichtungen schwierig zu fertigen. Des Weiteren müssen die Ringdichtungen als Reibdichtungen sorgfältig angepasst und auf Dichtigkeit geprüft werden. Zusätzlich ist diese Bauweise relativ groß.

Der vorliegenden Erfindung liegt die Aufgabe zugrunde, ein alternatives Kathetersystem der eingangs geschilderten Art bereit zu stellen. Die geschilderten Nachteile des Standes der Technik sollen dabei weitestgehend vermieden werden.

Die gestellte Aufgabe wird durch ein Kathetersystem mit den Merkmalen des unabhängigen Anspruches 1 gelöst. Weitere vorteilhafte Ausgestaltungen der Erfindung werden in den abhängigen Ansprüchen aufgeführt.

Erfindungsgemäß weist das äußere zweite Lumen eine Strömungsverbindung mit mindestens einem ersten Ballon auf und der erste Ballon ist derart mit der Hülle verbunden, dass eine Änderung des fluidgefüllten Volumens des ersten Ballons eine Bewegung der Hülle induziert. Der Grundgedanke der Erfindung besteht darin, ein oder mehrere Ballone zur Bewegung der Hülle und damit zur Freilegung des funktionalen Bauteils zu nutzen.

Die Verwendung und die Herstellung von Ballonkathetern sind im Stand der Technik etabliert. Gemäß der vorliegenden Erfindung wird jedoch nicht ein Stent mittel Expansion eines Ballons expandiert und in die Gefäßwand gedrückt. Gemäß der vorliegenden Erfindung wird über die Expansion eines oder mehrerer Ballone die Hülle von dem funktionalen Bauteil weg bewegt, so dass seine Funktion im Körpergefäß ausüben kann. Die Kraftübertragung zur Freisetzung des selbstexpandierenden Stents erfolgt somit hydraulisch über die Expansion mindestens eines Ballons.

Die vorliegende Erfindung weist daher verschiedene Vorteile auf. Zum einen erfolgt die Kraftübertragung zur Freisetzung des funktionalen Bauteils hydraulisch. Der oder die Ballone können vom Behandler einfach mit Fluid beaufschlagt werden und bewegen dadurch die Hülle vom funktionalen Bauteil weg. Die Freisetzung des funktionalen Bauteils erfolgt so ohne großen Kraftaufwand durch den Behandler, wodurch die Präzision der Freisetzung deutlich verbessert wird. Zum anderen verwendet die vorliegende Erfindung eine einfach und etablierte Technik zur hydraulischen Freisetzung des funktionalen Bauteils. Komplizierte Dichtungen, eine massive und unflexible Bauform wie im Stand der Technik sind durch die vorliegende Erfindung unnötig.

Unter einem funktionalen Bauteil wird im Rahmen der Erfindung ein Element im distalen Bereich des Katheters verstanden, welches im Körpergefäß eine bestimmte Funktion wahrnehmen soll. Ein derartiges funktionales Bauteil kann beispielsweise ein Implantat sein, welches über ein Kathetersystem in das Körpergefäß eingebracht und implantiert wird. Das Implantat, wie beispielsweise ein Stent, nimmt dann im Körpergefäß seine Funktion, beispielsweise seine Stützfunktion, wahr. Als funktionale Bauteile im Sinne der Erfindung kommen neben Stents, insbesondere selbstexpandierenden Stents, auch expandierbare Ballone, bildgebende Instrumente wie Ultraschallköpfe oder Miniaturkameras oder sonstige Instrumente wie Filter in Frage. Ein funktionales Bauteil im Sinne der Erfindung befindet sich jedoch immer im distalen Bereich des Katheters und ist bei Einführung und Platzierung im Körpergefäß mit einer beweglichen Hülle überdeckt. Das funktionale Bauteil ist dabei nicht zwingend Teil des Katheters sondern kann auch auf diesem angeordnet oder angebracht sein.

Unter einer beweglichen Hülle wird im Rahmen dieser Erfindung jede Art rohrförmiges Bauteil verstanden, welches geeignet ist, den selbstexpandierenden Stent zu umschließen. Im einfachsten Fall ist die bewegliche Hülle ein Rohrstück, welches mindestens so lang wie der selbstexpandierende Stent ist. Die bewegliche Hülle kann dabei sowohl aus Vollmaterial als auch mit Aussparungen (zum Beispiel Schlitzen) versehen sein. Ein rohrförmiges Gitter ist als bewegliche Hülle ebenfalls möglich, wenn eine hinreichende Steifigkeit gegeben ist.

Gemäß einer bevorzugten Ausführungsform der Erfindung ist der erste Ballon mit dem Teil der Hülle verbunden, der über die Länge des funktionalen Bauteils hinaus geht, wenn das Kathetersystem in dem bestimmungsgemäßen Zustand ist, in dem der Katheter in das Körpergefäß eingeführt wird.

Die bewegliche Hülle ist länger als das zugehörige funktionale Bauteil und überdeckt dieses vorteilhafterweise vollständig, wenn der Katheter in das Körpergefäß eingeführt wird. Durch die Hülle wird sichergestellt, dass das funktionale Bauteil an seiner Position bleibt. Ferner wird durch die bewegliche Hülle sichergestellt, dass bei Einführung des Katheters in das Körpergefäß das funktionale Bauteil nicht beschädigt oder in seiner Funktion beeinträchtigt und vor allem das Körpergefäß nicht durch die Einführung des funktionalen Bauteils verletzt wird. Der über das funktionale Bauteil hinausragende Teil der beweglichen Hülle ist in dieser Ausgestaltung mit dem Ballon in geeigneter Weise verbunden. Wird hier das Ballonlumen (das Innere des ersten Ballon oder der ersten Ballone) mit Fluid beaufschlagt und damit expandiert, wird eine Bewegung der Hülle derart induziert, dass die Überdeckung des funktionalen Bauteils durch die Hülle reduziert wird. Das funktionale Bauteil wird dadurch freigelegt.

Vorteilhafterweise wird der Ballon mit dem Teil der Hülle verklebt oder verschweißt, der über die Länge des funktionalen Bauteils hinaus geht, wenn das Kathetersystem in dem bestimmungsgemäßen Zustand ist, in dem der Katheter in das Körpergefäß eingeführt wird. Als Kleber geeignet sind hier beispielsweise handelsübliche Sekundenkleber und andere reaktive Klebstoffe, welche bevorzugt über eine chemische Reaktion aushärten. Diese Aushärtung kann mittels UV Bestrahlung oder Oberflächen und Umgebungskontakt initiiert werden, bzw. auf einem 2 Komponenten System beruhen welches thermisch beschleunigt härtbar ist. Diese Kleber sind beispielsweise Acrylate, Cyanacrylat, Epoxy Kleber oder Polyurethane.

Bevorzugt ist die Hülle entlang der Katheterachse beweglich.

Unter der Katheterachse wird im Rahmen der Erfindung die zentrale Längsachse des Katheters verstanden. Im Falle eines Over-the-Wire Katheters mit einer koaxialen Anordnung des ersten inneren Lumens mit dem Führungsdraht und Innenschaft mit umgebenden zweiten äußeren Lumen zur Fluidbeaufschlagung des Ballons und Außenschaft ist die Katheterachse die Längsachse des ersten inneren Lumens. Der Führungsdraht verläuft bei einem derartigen Katheter auf der Katheterachse.

In dieser Ausgestaltung der Erfindung ist die Hülle entlang der Katheterachse beweglich und wird entsprechend bei Freisetzung des Stents nach proximal oder distal durch die Fluidbeaufschlagung des ersten Ballons entlang der Katheterachse verschoben. Die Bewegung entlang der Katheterachse ist auch eine Bewegung entlang des Körpergefäßes beim Freisetzen des Stents. Entsprechend ist in dieser Ausgestaltung sicher gestellt, dass das Körpergefäß durch die Bewegung der Hülle beim Freisetzen des Stents keinen Schaden nehmen kann.

Bevorzugt ist das funktionale Bauteil ein Stent, insbesondere ein selbstexpandierender Stent, oder ein expandierbarer dritter Ballon, insbesondere ein expandierbarer dritter Ballon mit einer Beschichtung.

Unter einem selbstexpandierenden Stent wird im Rahmen der Erfindung jede Art von Implantaten verstanden, die aus einem Formgedächtnismaterial, insbesondere einer metallischen Formgedächtnislegierung (z.B. Nitinol) sind. Insbesondere werden darunter selbstexpandierende Stents verstanden, wie sie zur Behandlung einer Stenose in einem Körpergefäß verwendet werden. Die vorliegende Erfindung eignet sich auch insbesondere für selbstexpandierende Stents mit künstlichen Herzklappen.

Unter einem expandierbaren Ballon wird im Rahmen der Erfindung jede Art von Ballon verstanden, die durch Beaufschlagung mit einem Fluid expandiert wird.

In dieser bevorzugten Ausgestaltung der Erfindung kommen die Vorteile der Erfindung besonders zum Tragen. Die bewegliche Hülle überdeckt den selbstexpandierenden Stent vorteilhafterweise vollständig, wenn der Katheter in das Körpergefäß eingeführt wird. Durch die Hülle wird sichergestellt, dass der Stent auf den Schaft gepresst bleibt, d.h. auf den Schaft "gecrimpt". Dadurch wird gewährleistet, dass der Durchmesser des Kathetersystems klein genug ist, dass das Kathetersystem auch in kleine gewundene Körpergefäße eingeführt und vorgeschoben werden kann. Durch die Bewegung der Hülle wird die Überdeckung des selbstexpandierenden Stents mit der Hülle reduziert, wodurch die Rückhaltekraft auf den Stent entfällt und der Stent expandiert und somit freigesetzt wird.

Ähnliche Vorteile ergeben sich, wenn das funktionelle Bauteil ein Stent oder ein expandierbarer Ballon ist und diese mit einer Wirkstoff enthaltenden Beschichtung versehen sind. Die bewegliche Hülle stellt sicher, dass die Wirkstoff enthaltende Beschichtung nicht bei Einführung des Katheters beschädigt wird, sie schützt die wirkstofflialtige Schicht. Nach Positionierung wird die Hülle derart bewegt, dass der beschichtete Stent oder beschichtete Ballon nicht mehr von der Hülle überdeckt wird und der Wirkstoff direkt am gewünschten Ort freigesetzt wird.

In einer bevorzugten Ausgestaltung der Erfindung ist das funktionale Bauteil ein selbstexpandierender Stent. Besonders bevorzugt ist der selbstexpandierende Stent mittels eines proximalen Anschlags auf dem inneren Schaft positioniert bzw. mit diesem verbunden. In dieser Ausgestaltung der Erfindung wird zum einen sichergestellt, dass der Stent auch bei Einführen und Vorschieben des Katheters in das Körpergefäß an seiner Position im Katheter verbleibt. Der Anschlag kann dabei vorteilhafterweise aus röntgensichtbaren Material sein, so dass der Behandler die Position des Stents jederzeit bei Einbringen und Vorschieben des Katheters und insbesondere bei Freisetzen des Stents optimal kontrollieren kann. Insbesondere ist diese Ausgestaltung der Erfindung in Kombination mit einer nach proximal beweglichen Hülle vorteilhaft. Durch die proximale Bewegung der Hülle beim Freisetzen wirkt durch die Reibung eine Kraft in Richtung proximal auf den Stent. Dieser ist in seiner proximalen Bewegung jedoch durch den Anschlag gehindert. Eine distale Kraft wirkt in dieser Ausgestaltung der Erfindung nicht auf den Stent. Diese Ausgestaltung der Erfindung sichert so die maximale Genauigkeit bei der Freisetzung und Platzierung des Stents im Körpergefäß. In dieser Ausgestaltung der Erfindung wird die Hülle durch die Expansion des Ballons nach proximal über den Stent zurückgezogen.

In einer besonders bevorzugten Ausgestaltung der Erfindung ist die Hülle, bevorzugt über einen Zwischenschaft, mit mindestens einem zweiten Ballon verbunden, wobei der Katheter zumindest im distalen Bereich ein drittes äußeres Lumen aufweist, wobei das dritte äußere Lumen eine Strömungsverbindung mit dem zweiten Ballon aufweist.

Unter einem Zwischenschaft wird im Rahmen dieser Anmeldung jede vorrichtungsseitige Verbindung zwischen der beweglichen Hülle und dem zweiten Ballon verstanden, die geeignet ist eine hinreichende Kraftübertragung vom zweiten Ballon auf die bewegliche Hülle zu gewährleisten und eine Bewegung der Hülle durch die Änderung des zweiten Ballonlumens zu vermitteln. Unter dem zweiten Ballonlumen wird hier analog das Innere des zweiten Ballons oder der zweiten Ballone verstanden. Im einfachsten Fall besteht der Zwischenschaft aus dem gleichen Material wie Innen- und/oder Außenschaft, hat aber bevorzugt eine etwas größere Wandstärke und oder Festigkeit und oder Knickfestigkeit.

Bevorzugt ist dabei der erste Ballon in Richtung der Katheterachse von dem zweiten Ballon beabstandet.

Diese Ausgestaltung der Erfindung ist besonders für die Implantation von selbstexpandierenden Stents mit einer künstlichen Herzklappe geeignet. Diese Ausgestaltung mit einem ersten und einen zweiten Ballon oder einer ersten Gruppe und einer zweiten Gruppe von Ballonen, die insbesondere entlang der Katheterachse voneinander beabstandet sind, erlaubt die gesteuerte Bewegung der Hülle sowohl in distale als auch in proximale Richtung. Die Position der Hülle kann dabei in einfacher Weise und ohne mechanische Kraftaufwendung durch den Behandler präzise gesteuert werden.

Der erste und der zweite Ballon sind dabei zweckmäßigerweise so angeordnet, dass eine Fluidbeaufschlagung des einen verbunden mit einer Entlüftung des anderen eine Bewegung der Hülle in distaler Richtung und die Fluidbeaufschlagung des zweiten in Verbindung mit einer Entlüftung des ersten eine Bewegung der Hülle in proximaler Richtung induziert. Derart kann die Bewegung und Position der Hülle durch die Fluidbeaufschlagung und Entlüftung vom ersten und zweiten Ballon gesteuert werden. Werden sowohl der erste als auch der zweite Ballon mit dem gleichen Druck mit Fluid beaufschlagt, ändert sich die Position der beweglichen Hülle nicht. Wird der Druck bzw. das Volumen in dem distaler angeordneten Ballon erhöht und im proximalen verringert, wird entsprechend eine Bewegung der Hülle in proximaler Richtung induziert. Analog bewirkt eine Druck- bzw. Volumenerhöhung im proximaler angeordneten Ballon und eine Druck- bzw. Volumenverringerung im distalen eine Bewegung in distaler Richtung.

Der oder die zweiten Ballon/e sind dabei entweder direkt mit der beweglichen Hülle oder über den Zwischenschaft mit der beweglichen Hülle verbunden.

Wie bereits ausgeführt, kann in dieser Ausgestaltung der Erfindung die bewegliche Hülle hydraulisch sowohl nach proximal als nach distal bewegt werden. In der einen Richtung wird sie entsprechend von dem selbstexpandierenden Stent weg bewegt und setzt ihn entsprechend frei. Durch die Bewegung der Hülle in die entgegengesetzte Richtung kann der selbstexpandierende Stent wieder eingefangen werden.

Bevorzugt ist die bewegliche Hülle aus festen und feuchtigkeitsunempfindlichen Polymeren wie beispielsweise Polyamid (10-) 12 , Trogamid, Cristamid oder Aramid, Polyester wie Polyethylenterephthalat (PET) und/oder Polyimid hergestellt. Hierbei kann es auch zweckmäßig sein, verschiedene Materialien miteinander zu vermischen, um eine geeignete Mischung aus hoher Festigkeit und guten Gleiteigenschaften zu erreichen. Ebenso zweckmäßig ist die Co-Extrusion der genannten Materialien bzw. das Verstärken mittels Metalldrähten (z.B. Coil und Braid) um spezifische Eigenschaften wie Oberflächenhärte und Reibung an Innenflächen zu beeinflussen und genügend Gesamtfestigkeit und Flexibilität zur Verfügung zu stellen.

Insbesondere wenn das funktionale Bauteil ein selbstexpandierender Stent ist, kann es zweckmäßig sein, die bewegliche Hülle eher als Gitterstruktur aus zu gestalten.

Bevorzugt ist der Zwischenschaft aus einem ähnlichen Material wie die restlichen Katheterschäfte, weist aber bevorzugt eine leicht höhere Festigkeit und oder Knickfestigkeit aus. Zweckmäßigerweise ist der Katheter aus Polyethylen (PE), Vinylpolymer, Polyester wie beispielsweise PET, Polyamid wie beispielsweise Polyetherblockamide (PEBA) und/oder Polyimid oder Polyetheretherketon (PEEK). Auch hier können zweckmäßigerweise geeignete Materialien co-extrudiert bzw. verstärkt werden, um sowohl gute Gleiteigenschaften bei hinreichender Steifigkeit zu erhalten.

Besonders bevorzugt ist der selbstexpandierende Stent zur Behandlung einer Stenose in einem Köpergefäß geeignet. Der selbstexpandierende Stent trägt vorteilhafterweise eine künstliche Herzklappe und/oder ist zum Einsetzen an der physiologischen Position einer der Herzklappen geeignet.

Die Vorteile der vorliegenden Erfindung kommen insbesondere zum Tragen, wenn der selbstexpandierende Stent eine künstliche Herzklappe aufweist und/oder an der physiologische Position einer Herzklappe eingesetzt wird.

Bei Herzklappen, wie beispielsweise der Aortenklappe, kann es durch Ablagerungen (Verkalkungen) zu Fehlfunktionen kommen. In diesem Fall schließt oder öffnet die Klappe nicht mehr vollständig. In einem derartigen Fall kann eine künstliche Herzklappe eingesetzt werden. Dazu wird zumeist ein selbstexpandierender Stent an die physiologische Position der natürlichen Herzklappe gesetzt. Dieser Stent trägt die künstliche Herzklappe, die die Funktion der natürlichen Klappe übernimmt.

Bei einem derartigen Verfahren ist die exakte Positionierung der Stents, insbesondere des Stents mit der künstlichen Herzklappe, entscheidend. Hier kommen die Vorteile der vorliegenden Erfindung besonders zum Tragen. Durch die hydraulische Kraftübertragung bei der Bewegung der Hülle durch Expansion eines Ballons lässt sich die Freisetzung des selbstexpandierenden Stents sehr genau und präzise steuern. Insbesondere die Ausgestaltung der Erfindung mit mindestens einem ersten Ballon und mindestens einem zweiten Ballon erlaubt eine sehr gute Steuerung der Positionierung durch den Behandler. Durch geeignete Röntgenmarker, beispielsweise am proximalen Anschlag, lässt sich die Position des selbstexpandierenden Stents permanent während der Freisetzung überwachen. Durch das Zurückschieben der Hülle durch den zweiten Ballon ergibt sich zusätzlich die Möglichkeit den teilweise freigesetzten Stent bei Fehlpositionierung wieder einzufangen und die Positionierung zu korrigieren.

In einer anderen bevorzugten Ausgestaltung der Erfindung weist der expandierbare dritte Ballon eine Beschichtung auf, die eine therapeutisch wirksame Substanz, insbesondere eine therapeutisch wirksame Substanz zur Behandlung einer Stenose in einem Körpergefäß, enthält. In dieser Ausgestaltung der Erfindung ist das funktionale Bauteil ein wirkstoffbeschichteter Ballon. Der Wirkstoff soll erst am Behandlungsort bei Expansion des Ballons freigesetzt werden. Die bewegliche Hülle verhindert entsprechend eine Schädigung der Beschichtung bei Einbringen des Katheters in das Körpergefäß und vermeidet eine Schädigung des Patienten durch falsch freigesetzten Wirkstoff. Auch in dieser Ausgestaltung wird die bewegliche Hülle durch Fluidbeaufschlagung des ersten Ballons und/oder zweiten Ballons in Bezug auf den expandierbaren dritten Ballon bewegt. Der Behandler kann die Hülle analog zur Applikation des Wirkstoffes durch Fluidbeaufschlagung und dadurch hervorgerufene Druckveränderungen bewegen.

Die vorliegende Erfindung weist insbesondere den Vorteil einer genauen Positionierung eines selbstexpandierenden Stents auf. Ferner wird durch die hydraulische Kraftübertragung eine einfache Handhabung des Kathetersystems bei Freilegung des funktionalen Bauteils gewährleistet. Zusätzlich zeichnet sich das erfindungsgemäße Kathetersystem durch eine einfache und erprobte Fertigung durch die Verwendung von expandierbaren Ballonen zur Bewegung der Hülle aus. Durch die Vermeidung von Reibdichtungen und Kolben wie bei einem Kathetersystem nach dem Stand der Technik lässt sich das erfindungsgemäße Kathetersystem kleiner und geringerer Steifigkeit ausführen. Entsprechend kann das erfindungsgemäße Kathetersystem auch bei kleinen und gewundenen Körpergefäßen eingesetzt werden.

Die vorliegende Erfindung soll im Folgenden anhand des in der Figur dargestellten Ausführungsbeispieles, bei dem das funktionale Bauteil ein selbstexpandierender Stent ist, näher erläutert werden.

Es zeigen:
- Figur 1: ein Ausführungsbeispiel eines erfindungsgemäßen Kathetersystem im bestimmungsgemäßen Zustand, in dem der Katheter in das Körpergefäß eingeführt wird,
- Figur 2: das Ausführungsbeispiel nach Figur 1 bei teilweise freigesetzten Stent und
- Figur 3: das Ausführungsbeispiel nach Figur 1 bei vollständig freigesetzten Stent.

Dabei wird in den Figuren nur der distale Teil des Ausführungsbeispieles dargestellt.

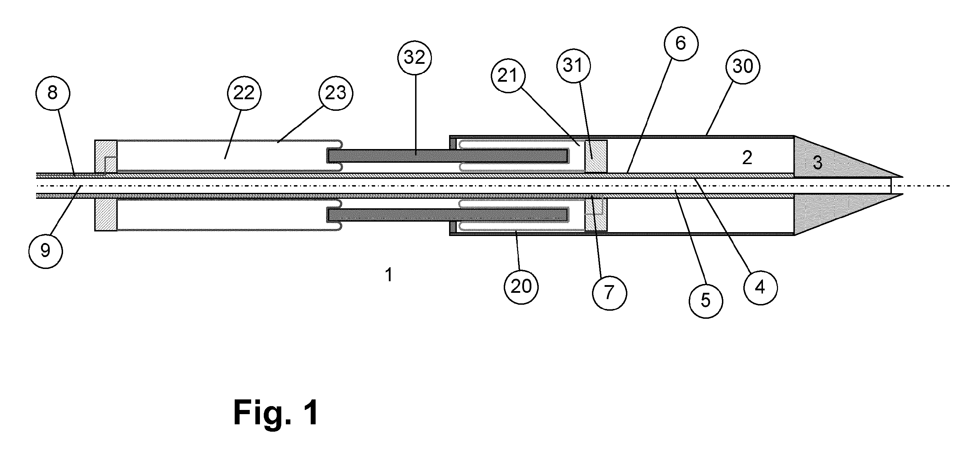

Figur 1 zeigt ein Ausführungsbeispiel eines erfindungsgemäßen Kathetersystems in dem bestimmungsgemäßen Zustand, in dem der Katheter 1 in das Körpergefäß eingeführt wird. Der selbstexpandierende Stent, beispielsweise ein selbstexpandierender Stent mit einer künstlichen Herzklappe, befindet sich in dem Hohlraum 2 und wird vollständig von der Hülle 30 bedeckt. Die Hülle 30 verhindert dass der Stent expandiert. Der Stent bleibt im komprimierten Zustand und hat somit einen leicht kleineren Durchmesser wie der Katheter 1 selbst und kann so mit dem Katheter 1 in das Körpergefäß eingebracht werden.

Der Katheter 1 hat einen inneren Schaft 4, welcher das erste innere Lumen 5 umgibt. Das innere Lumen 5 dient zumeist zur Aufnahme des Führungsdrahtes. Der Katheterschaft 6 selbst ist als Schaft mit drei verschiedenen Lumen ausgestaltet, dem dargestellten inneren Lumen 5 für den Führungsdraht sowie den äußeren zweiten Lumen 7 und dem äußeren dritten Lumen 8 (beide nicht im Detail dargestellt). Beide Lumen 7 und 8 können koaxial oder parallel zur Katheterachse 9 angeordnet sein.

Das zweite äußere Lumen 7 weist eine Strömungsverbindung mit dem Inneren 21 des ersten Ballons 20 auf, wodurch der erste Ballon 20 mit Fluid beaufschlagt werden kann.

Der Zwischenschaft 32 dient der Bewegung der Hülle 30 und ist an seinem proximalen Ende mit einem zweiten Ballon 23 verbunden. Das Innere 22 des zweiten Ballons 23 ist über das dritte äußere Lumen 8 in Strömungsverbindung mit einer zweiten Fluidquelle und kann separat vom ersten Ballon 20 mit Fluid beaufschlagt werden.

Der selbstexpandierende Stent (nicht dargestellt) befindet sich im Raum 2, wobei die Position des Stents bei Einführung des Katheters in das Köpergefäß durch die bewegliche Hülle 30 über den Stent und den proximalen Anschlag 31 exakt definiert ist. Im bestimmungsgemäßen Zustand, in dem das Kathetersystem in das Köpergefäß eingeführt ist, erstreckt sich die Hülle 30 über den gesamten Bereich des Katheters vom Anschlag 31 bis zur Spitze 3 des Katheters.

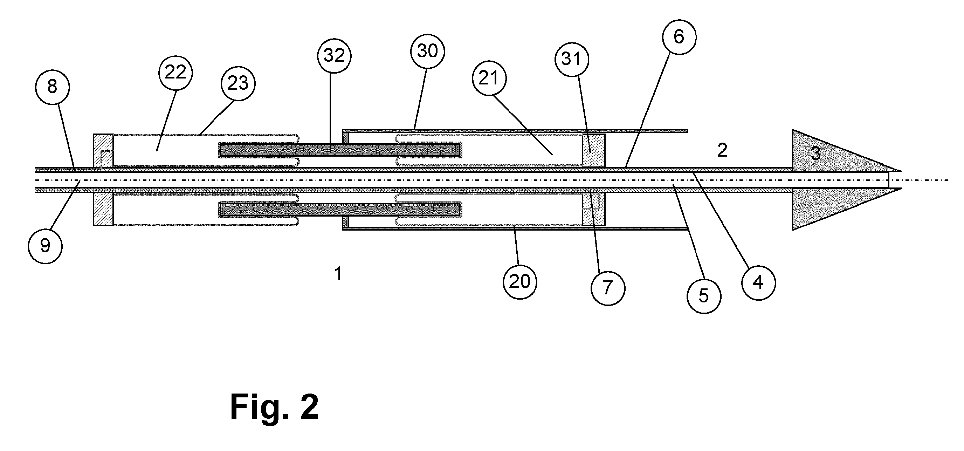

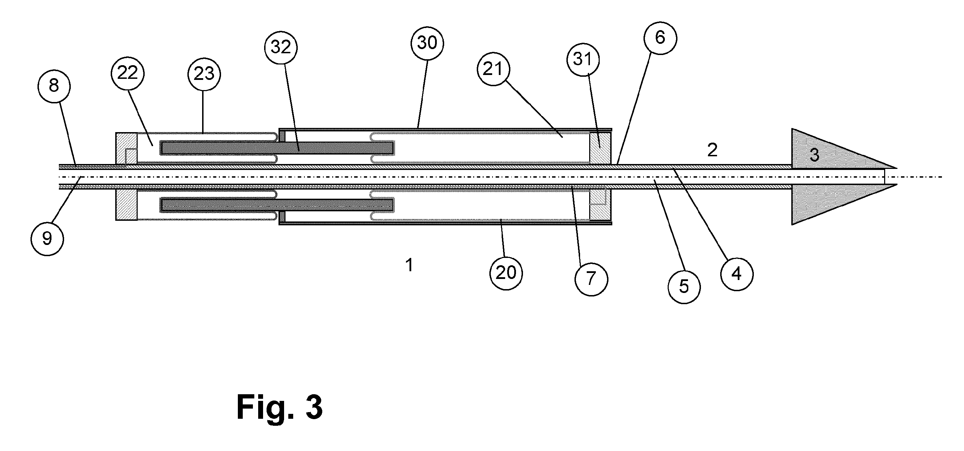

Das Freisetzen des selbstexpandierenden Stents im Körpergefäß wird im Folgenden anhand der Figuren 2 und 3 beschrieben. Die beiden Figuren 2 und 3 zeigen die gleiche Ausgestaltung des erfindungsgemäßen Kathetersystems wie Figur 1. Gleiche Teile wurden mit gleichen Bezugsziffern versehen.

Bei Einführung des Katheters 1 in das Körpergefäß wird der zweite Ballon 23 mit einem deutlich höheren Druck als der erste Ballon 20 beaufschlagt. Dadurch wird die bewegliche Hülle 30 über den Zwischenschaft 32 zum distalen Ende des Katheters gedrückt. Die bewegliche Hülle 30 überdeckt so den selbstexpandierenden Stent vollständig und hält ihn in seiner komprimierten Form auf dem Katheter. Ein Zurückschieben der Hülle durch Reibungskräfte des Katheters mit den Wänden des Körpergefäßes oder dem hämostatischen Ventil der Einführschleuse bei Einführung des Katheters wird durch den Gegendruck im zweiten Ballon 23 vermieden. Derart kann das erfindungsgemäße Kathetersystem sicher in das Körpergefäß eingeführt werden. Im Normalfall ist bei Einführung des Kathetersystems in das Körpergefäß der Druck im Inneren 21 des ersten Ballons 20 nahezu 0, das Innere 21 des ersten Ballons 20 ist kaum mit Fluid gefüllt.

Wenn sich das Kathetersystem im Körpergefäß befindet, kann die Position des selbstexpandierenden Stents laufend durch die röntgensichtbare Spitze 3, Röntgenmarkern in der beweglichen Hülle 30 und/oder im Anschlag 31 und/ oder in der Spitze 3 und/ oder am/im Innenschaft 4 kontrolliert werden. Befindet sich der selbstexpandierende Stent an der gewünschten Position kann mit der Freisetzung des Stents begonnen werden. Dazu wird das Innere 21 des ersten Ballons 20 Fluid beaufschlagt, während gleichzeitig der Druck im Inneren 22 des zweiten Ballons 23 reduziert wird. Beide Ballone 20, 23 sind über den Zwischenschaft 32 mit der beweglichen Hülle 30 verbunden. Die Druckerhöhung im Inneren 21 des ersten Ballons 20 führt zur Vergrößerung des Volumens. Durch das vergrößerte Volumen 21 des ersten Ballons 20 wird die bewegliche Hülle 30 über das Zwischenschaft 32 nach proximal bewegt.

Der Gegendruck und das Volumen des zweiten Ballons 23 werden dabei reduziert. Durch den gleichzeitigen Druckaufbau im ersten Ballon 20 und Druckabbau im zweiten Ballon 23 ergibt sich eine sehr kontrollierte Bewegung der Hülle 30. Entsprechend kann der selbstexpandierende Stent aus dem Raum 2 sehr kontrolliert freigesetzt werden.

Figur 2 zeigt einen entsprechenden Zwischenzustand, wo im Vergleich zu Figur 1 der Druck im Inneren 21 des ersten Ballons 20 gestiegen und der Druck im Inneren 22 des zweiten Ballons 23 reduziert wurde. Bei dem in Figur 2 gezeigten Zustand sind der Druck im Inneren 21 des ersten Ballons 20 und der Druck im Inneren 22 des zweiten Ballons 23 gleich. Die Hülle 30 hat sich entsprechend kontrolliert nach proximal bewegt und den selbstexpandierenden Stent zur Hälfte freigesetzt.

Figur 3 zeigt den Zustand der vollständigen Freisetzung des selbstexpandierenden Stents. Die Druckverhältnisse im Inneren 21, 22 der beiden Ballon 20, 23 sind im Vergleich zum Zustand in Figur 1 invertiert. Der zweite Ballon 23 ist drucklos, der erste Ballon 20 ist mit Fluid beaufschlagt und voll expandiert. Entsprechend sind Zwischenschaft 32 und bewegliche Hülle 30 maximal nach proximal zurück bewegt.

Das erfindungsgemäße Kathetersystem bietet hier auch die Möglichkeit des erneuten Einfangens und der Repositionierung des selbstexpandierenden Stents. Hierbei müssen nur die Druckverhältnisse im Inneren 21, 22 der Ballone 20, 23 erneut umgekehrt werden. Der Druck im Inneren 22 des zweiten Ballons 23 wird durch Fluidbeaufschlagung wieder vergrößert, während der Druck im Inneren 21 des ersten Ballons 20 wieder verringert wird. Der Zustand des Kathetersystems aus Figur 3 geht analog über den Zustand aus Figur 2 wieder in den Zustand des Kathetersystems wie in Figur 1 dargestellt über. Dabei kann der Stent jedoch nur solange wieder eingefangen werden, wie er auch noch auf dem Katheter gehalten wird (sich zum Beispiel noch zu einem Drittel unter der Hülle 30 befindet).

Durch die Steuerung der beiden Drücke bzw. Volumen im Inneren 21, 22 bzw. des Druckverhältnisse der Ballone 20, 23 lässt sich präzise jede beliebige Position der Hülle einstellen. Die Kraft mit der die Hülle 30 bewegt wird, beispielsweise beim Zurückschieben über den schon expandierten Stent, lässt sich durch die Querschnittsfläche des Zwischenschaftes 32 bestimmen. Die Kraft ergibt sich direkt aus dem Produkt der Druckdifferenz und der Querschnittsfläche des Zwischenschaftes 32.

## Patentansprüche

1. Kathetersystem umfassend einen Katheter (1) mit einem funktionalen Bauteil im distalen Bereich,
- wobei das Kathetersystem zum Einbringen in ein Körpergefäß geeignet ist,
- wobei der Katheter (1) an seinem distalen Bereich eine bewegliche Hülle (30) aufweist, deren Durchmesser zumindest dem Durchmesser des funktionalen Bauteils entspricht, und deren Länge größer als die Länge funktionalen Bauteils ist,
- wobei die Hülle (30) das funktionale Bauteil überdeckt, wenn das Kathetersystem in dem bestimmungsgemäßen Zustand ist, in dem der Katheter (1) in das Körpergefäß eingeführt wird,
- wobei der Katheter (1) zumindest im distalen Bereich einen Schaft (6) mit einem ersten inneren Lumen (5) und ein äußeres zweites Lumen (7) aufweist,
**dadurch gekennzeichnet, dass**
das äußere zweite Lumen (7) eine Strömungsverbindung mit mindestens einem ersten Ballon (20) aufweist und der erste Ballon (20) mit derart mit der Hülle (30) verbunden ist, das eine Änderung des fluidgefüllten Volumens (21) des ersten Ballons (20) eine Bewegung der Hülle (30) induziert.

2. Kathetersystem nach Anspruch 1, **dadurch gekennzeichnet, dass** der erste Ballon (20) mit dem Teil der Hülle (30) verbunden ist, der über die Länge des funktionalen Bauteils hinaus geht, wenn das Kathetersystem in dem bestimmungsgemäßen Zustand ist, in dem der Katheter (1) in das Körpergefäß eingeführt wird.

3. Kathetersystem nach Anspruch 1 oder 2, **dadurch gekennzeichnet, dass** die Hülle (30) entlang der Katheterachse (9) beweglich ist.

4. Kathetersystem nach einem der vorangegangenen Ansprüche, **dadurch gekennzeichnet, dass** das funktionale Bauteil ein Stent, insbesondere ein selbstexpandierender Stent, oder ein expandierbarer dritter Ballon, insbesondere ein expandierbarer dritter Ballon mit einer Beschichtung, ist.

5. Kathetersystem nach Anspruch 4, **dadurch gekennzeichnet, dass** der selbstexpandierende Stent mittels eines proximalen Anschlags (31) auf dem Schaft (6) positioniert und/oder mit diesem verbunden ist.

6. Kathetersystem nach einem der vorangegangenen Ansprüche, **dadurch gekennzeichnet, dass** die Hülle (30), bevorzugt über einen Zwischenschaft (32), mit mindestens einem zweiten Ballon (23) verbunden ist, wobei der Katheter (1) zumindest im distalen Bereich ein drittes äußeres Lumen (8) aufweist, wobei das dritte äußere Lumen (8) eine Strömungsverbindung mit dem zweiten Ballon (23) aufweist.

7. Kathetersystem nach einem der vorangegangenen Ansprüche, **dadurch gekennzeichnet, dass** der erste Ballon (20) in Richtung der Katheterachse (9) von dem zweiten Ballon (23) beabstandet ist.

8. Kathetersystem nach einem der Ansprüche 4 bis 7, **dadurch gekennzeichnet, dass** der selbstexpandierende Stent zur Behandlung einer Stenose in einem Köpergefäß geeignet ist.

9. Kathetersystem nach einem der Ansprüche 4 bis 8, **dadurch gekennzeichnet, dass** der selbstexpandierende Stent eine künstliche Herzklappe trägt und/oder zum Einsetzen an der physiologischen Position einer der Herzklappen geeignet ist.

10. Kathetersystem nach Anspruch 4 bis 7, **dadurch gekennzeichnet, dass** der expandierbare dritte Ballon eine Beschichtung aufweist, die eine therapeutische wirksame Substanz, insbesondere eine therapeutisch wirksame Substanz zur Behandlung einer Stenose in einem Körpergefäß, enthält.
